# EUROPÄISCHE PATENTSCHRIFT

(11) **EP 2 965 771 B1**
(45) Veröffentlichungstag und Bekanntmachung des Hinweises auf die Patenterteilung: **09.08.2017**
(21) Anmeldenummer: 15172872.2
(22) Anmeldetag: 19.06.2015
(51) Int. Cl.: F16K 7/12, F16K 47/04, A61M 39/24, A61M 39/22, A61M 1/14

(54) **VENTIL FÜR EIN DIALYSEGERÄT UND DIALYSEGERÄT**
VALVE FOR A DIALYSIS MACHINE AND DIALYSIS MACHINE
VALVE POUR UN APPAREIL DE DIALYSE ET APPAREIL DE DIALYSE

(30) Priorität: 09.07.2014 DE 102014109639
(43) Veröffentlichungstag der Anmeldung: 13.01.2016
(73) Patentinhaber: B. Braun Avitum AG, 34212 Melsungen (DE)
(72) Erfinder: Mänz, Peter, 34212 Melsungen (DE); Wagner, André, 34117 Kassel (DE); Felski, Katharine, 34212 Melsungen (DE); Rössler, Rudolf, 34286 Spangenberg (DE); Riemann, Martin, 36211 Alheim (DE); Bröker, Björn, 34355 Staufenberg (DE); Osztódi, Tibor, 1031 Budapest (HU)
(74) Vertreter: Winter, Brandl, Fürniss, Hübner, Röss, Kaiser, Polte - Partnerschaft mbB

(56) Entgegenhaltungen:
- WO-A1-02/056992
- WO-A2-2009/055639
- DE-T2- 69 328 271
- DE-T2- 69 819 715
- JP-A- H05 146 506
- US-A- 5 009 775

## Beschreibung

Die Erfindung geht aus von einem Ventil, insbesondere einem Druckausgleichsventil, für ein Dialysegerät. Des Weiteren betrifft die Erfindung ein Dialysegerät mit einem derartigen Ventil.

In der EP 0 597 817 ist ein derartiges Dialysegerät offenbart. Hierbei wird frische Dialysierflüssigkeit über eine Pumpe von einer Dialysierflüssigkeitsquelle zu einem Dialysator gefördert. Vom Dialysator fließt verbrauchte Dialysierflüssigkeit zu einer Dialysierflüssigkeitssenke. Damit frische und verbrauchte Dialysierflüssigkeit bilanziert werden können, ist eine Bilanzierungseinrichtung vorgesehen. Diese hat eine erste und zweite Bilanzierungskammer. In einer jeweiligen Bilanzierungskammer ist eine flexible Membran angeordnet, die jeweils zwei Zellen voneinander trennt. Die ersten Zellen der Bilanzierungskammern können unabhängig voneinander über Schaltventile mit frischer Dialysierflüssigkeit beschickt werden und es kann frische Dialysierflüssigkeit aus diesen entlassen werden. Die zweiten Zellen der Bilanzierungskammern können dagegen unabhängig voneinander über Schaltventile mit verbrauchter Dialysierflüssigkeit beschickt werden und es kann verbrauchte Dialysierflüssigkeit aus diesen entlassen werden. Zum Ausgleichen der Drücke ausgangsseitig der Zellen ist ein erster Ausgangsdruckausgleicher vorgesehen. Zum Ausgleichen der Drücke eingangsseitig der Zellen ist ein Eingangsdruckausgleicher vorgesehen. Nachteilig hierbei ist, dass mit den Druckausgleichern zwar Drücke korrigiert werden können, aber es nicht möglich ist, den Druck zu definieren, mit dem die die Dialysierflüssigkeit fördernden Pumpen beaufschlagt werden. Außerdem hat sich als nachteilig herausgestellt, dass die Druckausgleicher starke Vibrationen verursachen und eine hohe Geräuschemission aufweisen. Dies kann zum Einen eine Funktionsweise des Dialysegeräts negativ beeinflussen und zum anderen kann es unangenehm für einen mit dem Dialysegerät behandelten Patienten sein.

Dem gegenüber liegt der Erfindung die Aufgabe zugrunde ein Ventil zu schaffen, mit dem ein Druckausgleich in einem Dialysegerät erfolgen kann, wobei im Betrieb auf vorrichtungstechnisch einfache Weise eine geringe Geräuschemission und/oder geringe Vibrationen vorherrschen. Des weiteren liegt der Erfindung die Aufgabe zugrunde, ein Dialysegerät mit einem Ventil zu schaffen, das eine hohe Genauigkeit hinsichtlich einer Menge einer zu einem Dialysator zugeführten und abgeführten Dialysierflüssigkeit aufweist.

Die Aufgabe hinsichtlich des Ventils wird gelöst gemäß den Merkmalen des Anspruchs 1 und hinsichtlich des Dialysegeräts gemäß den Merkmalen des Anspruchs 10. Sonstige vorteilhafte Weiterbildungen der Erfindung sind Gegenstand weiterer Unteransprüche.

Erfindungsgemäß ist ein Ventil, insbesondere ein Druckausgleichsventil, insbesondere ein Pressure Follower Valve, für ein Dialysegerät vorgesehen. Dieses hat ein Ventilgehäuse in dem ein erster Strömungspfad für frische Dialysierflüssigkeit und ein zweiter Strömungspfad für verbrauchte Dialysierflüssigkeit ausgebildet ist. Ein jeweiliger Strömungspfad ist mit einem jeweiligen Eingangsanschluss und einem jeweiligen Ausgangsanschluss verbunden. Zum Ausgleichen oder Angleichen von Drücken der Strömungspfade ist in dem Ventilgehäuse ein Ausgleichselement vorgesehen. Über das Ausgleichselement stehen die Dialysierflüssigkeit eines jeweiligen Strömungspfads in Wirkverbindung. Vorteilhafter Weise ist das Ausgleichselement derart ausgestaltet, dass eine Vibration und/oder Geräuschemission reduziert ist oder vergleichsweise gering ist oder verhindert ist. Alternativ oder zusätzlich kann vorgesehen sein, dass dem Ausgleichselement ein Ventilsitz zugeordnet ist, um mit dem Ausgleichselement eine Größe eines Strömungsquerschnitts des - insbesondere zweiten - Strömungspfads einzustellen.

Es hat sich gezeigt, dass Vibrationen und Geräuschemission des Ventils deutlich reduziert oder verhindert werden können, wenn einfach dessen Geometrie angepasst wird. Es sind somit keine zusätzlichen Bauteile notwendig, um Vibrationen und Geräusche zu minimieren, sondern es wird bauraumsparend, vorrichtungstechnisch einfach und kostengünstig das Ausgleichselement angepasst. Die Verminderung der Vibrationen und Geräuschemissionen erfolgt vorzugsweise durch Optimieren einer Strömungsgeometrie des Ausgleichselements. Ist ein Ventilsitz vorgesehen, so kann auf einfache Weise effektiv ein Druckausgleich erfolgen.

Vorzugsweise ist das Ausgleichselement derart ausgestaltet und im Ventil angeordnet, dass eine an den Eingangsanschlüssen vorhandene Druckdifferenz zwischen den Strömungspfaden an den Ausgangsanschlüssen Null oder im Wesentlichen Null ist.

Bei dem Ausgleichselement handelt es sich vorzugsweise um eine Membran. Diese kann einerseits von frischer Dialysierflüssigkeit des ersten Strömungspfads und andererseits von verbrauchter Dialysierflüssigkeit des zweiten Strömungspfads beaufschlagt sein. Zur Verminderung oder Verhinderung der Vibrationen oder Geräuschemissionen hat die Membran einen Membranvorsprung, der insbesondere von Dialysierflüssigkeit bedeckt ist. Durch die Membran können innerhalb des Ventils frische Dialysierflüssigkeit und verbrauchte Dialysierflüssigkeit ohne direkten Kontakt in Wirkverbindung stehen, wobei beide Strömungspfade eine gleiche Flussleistung (ml/min) aufweisen können.

Der Membranvorsprung ist vorzugsweise vorrichtungstechnisch einfach einstückig mit der Membran ausgebildet. Die Membran besteht beispielsweise aus Silikon, wodurch die Membran sehr flexibel ist. Außerdem kann die Membran beispielsweise kostengünstig durch ein Spritzgussverfahren hergestellt sein.

Bevorzugterweise ist der Membranvorsprung mittig der Membran angeordnet.

Beispielsweise ist der Membranvorsprung auf derjenigen Membranseite der Membran angeordnet, die den zweiten Strömungspfad begrenzt und somit beispielsweise von verbrauchter Dialysierflüssigkeit beaufschlagt sein kann.

Die Membran kann symmetrisch ausgestaltet sein und insbesondere einen etwa kreisförmigen Umfang aufweisen. Umfangsseitig kann die Membran einen ringförmigen Keder aufweisen, über den sie im Ventilgehäuse befestigt ist. Der Keder kann zusätzlich als Dichtelement eingesetzt sein und somit eine Doppelfunktion erfüllen, nämlich die Membran im Ventilgehäuse zu fixieren und zusätzlich die Strömungspfade voneinander dichtend zu trennen. Somit sind kostengünstig keine weiteren Dichtelemente erforderlich.

Mit Vorteil ist in einer Membranseite, insbesondere in der Membranseite mit dem Membranvorsprung, eine Ringnut ausgebildet, die sich umlaufend um den Membranvorsprung erstreckt. Im Bereich der Ringnut hat die Membran eine verminderte Wandstärke, womit diese äußerst flexibel ist. In Radialrichtung der Membran gesehen kann die Ringnut beispielsweise zwischen dem Membranvorsprung und dem Keder ausgebildet sein.

In weiterer Ausgestaltung der Erfindung begrenzt die Membran auf Seiten des zweiten Strömungspfads eine Fluidkammer bzw. einen Fluidraum des Strömungspfads, in dem ein Kanalabschnitt des zweiten Strömungspfads mündet. Der Kanalabschnitt und die Membran sind dabei derart zueinander angeordnet, dass der Membranvorsprung in den Kanalabschnitt eintauchen kann. Der Membranvorsprung begrenzt somit zusammen mit dem Kanalabschnitt einen ringförmigen von Dialysierflüssigkeit durchströmbaren Fluidraum. Es hat sich gezeigt, dass dies effektiv zu einer Geräuschreduzierung und Verminderung der Vibrationen führen kann.

Vorzugsweise ist die Größe eines Strömungsquerschnitts des Kanalabschnitts abhängig von einer Position des Membranvorsprungs, womit die Größe des Strömungsquerschnitts durch den Membranvorsprung veränderbar ist. Steigt beispielsweise der Druck im ersten Strömungspfad und/oder vermindert sich der Druck im zweiten Strömungspfad, so kann der Strömungsquerschnitt durch den Membranvorsprung verkleinert werden. Umgekehrt kann bei einem Absinken des Drucks im ersten Strömungspfad und/oder eines Ansteigens des Drucks im zweiten Strömungspfad der Strömungsquerschnitt durch den Membranvorsprung vergrößert sein.

In weiterer Ausgestaltung der Erfindung ist denkbar, dass der zweite Strömungspfad von der Membran bei entsprechenden Druckverhältnissen vollständig verschlossen sein kann. Beispielsweise ist die Mündungsöffnung des Kanalabschnitts von einer Ventilsitzfläche des Ventilsitzes für die Membran umfasst. Durch die Ventilsitzfläche ist somit der Ventilsitz zum Verschließen des Kanalabschnitts für die Membran gebildet. Zum Anlegen an die Ventilsitzfläche hat die Membran vorzugsweise eine ringförmige Anschlagschulter, die sich in Umfangsrichtung um den Membranvorsprung erstrecken kann. Die Anschlagschulter ist beispielsweise in Radialrichtung gesehen zwischen dem Membranvorsprung und der Ringnut ausgebildet.

Eine Mündungsöffnung des Kanalabschnitts des zweiten Strömungspfads ist vorzugsweise koaxial zur Membran und somit koaxial zum Membranvorsprung ausgebildet. Strömt verbrauchte Dialysierflüssigkeit im zweiten Strömungspfad von der Fluidkammer über die Mündungsöffnung in den Kanalabschnitt, so hat sich gezeigt, dass zusammen mit der Membran dies zu vorteilhaften Strömungsbedingungen führt, die Vibrationen und/oder Geräuschemissionen der Membran vermindern oder verhindern.

Weiter vorteilhaft ist, wenn der Membranvorsprung kegelförmig oder kegelstumpfförmig ausgebildet ist und sich in einer Richtung weg von der Membran verjüngt. Ein Endabschnitt des Membranvorsprungs kann hierbei zusätzlich kugelsegmentförmig ausgestaltet sein. Der Membranvorsprung weist somit eine abgerundete Spitze auf.

Die Mündungsöffnung des Kanalabschnitts des zweiten Strömungspfads hat vorzugsweise eine etwa kegelstumpfförmige Innenmantelfläche, die sich in einer Richtung weg von der Membran verjüngt.

Durch die kegelstumpfförmige Ausgestaltung der Außenmantelfläche des Membranvorsprungs und der Innenmantelfläche der Mündungsöffnung ist ein durchströmbarer Ringraum begrenzt, dessen Strömungsquerschnitt abhängig von der Position des Membranvorsprungs sein kann. Vorzugsweise ist ein Anstellwinkel der kegelstumpfförmigen Innenmantelfläche der Mündungsöffnung und ein Anstellwinkel der kegelstumpfförmigen Außenmantelfläche des Membranvorsprungs jeweils derart gewählt, dass sich die Flächen hin in Richtung zur Membran voneinander entfernen. Vorzugsweise erstreckt sich die kegelstumpfförmige Innenmantelfläche der Mündungsöffnung ausgehend von der Ventilsitzfläche. Ist die Ventilsitzfläche von einer kegelstumpfförmigen Umfangsfläche umgriffen, die sich in Richtung weg von der Membran verbreitert, so führt dies zu einer genauen Definition der Ventilsitzfläche.

In weiterer Ausgestaltung der Erfindung mündet der Kanalabschnitt mit seiner Mündungsöffnung als Vorsprung in der Fluidkammer und begrenzt darin einen Ringraum, was strömungstechnisch äußerst vorteilhaft ist. Der Ringraum ist beispielsweise etwa rotationssymmetrisch ausgestaltet, wobei dessen Längsachse sich etwa koaxial zur Längsachse des Kanalabschnitts und/oder zur Längsachse der Membran erstrecken kann. Vorzugsweise ist der Kanalabschnitt mit dem zweiten Ausgangsanschluss verbunden und der Fluidraum mit dem zweiten Eingangsanschluss. Der zweite Eingangsanschluss ist über einen etwa in die Fluidkammer mündenden Verbindungskanal mit der Fluidkammer verbunden. Der Verbindungskanal mündet vorzugsweise radial oder tangential in den Fluidraum und ist vorzugsweise von der Membran maximal beabstandet.

Vom zweiten Strömungspfad, insbesondere von der Fluidkammer oder stromaufwärts der Fluidkammer (beispielsweise vom Verbindungskanal) kann ein Bypass-Kanal abzweigen. Dieser ist beispielsweise mit einem Bypass-Anschluss des Ventilgehäuses verbunden. Dieser Bypass-Kanal ist insbesondere dann erforderlich, wenn bei dem Dialysegerät eine Substitution oder Online-Infusionsbolusgabe vorgesehen ist. An dem Bypass-Anschluss kann ein Bypass-Ventil angeschlossen sein. Dieses ist vorzugsweise direkt mit dem Ventilgehäuse verbunden und kann somit mit diesem eine Einheit bilden. Mit dem Bypass-Ventil ist beispielsweise der Bypass-Kanal auf- und zusteuerbar. Ein Ventilelement des Bypass-Ventils ist hierbei insbesondere über einen elektromagnetischen Aktuator betätigbar.

Der erste Strömungspfad hat vorzugsweise ebenfalls eine Fluidkammer, die von einer Membranseite der Membran begrenzt ist.

Bevorzugter Weise hat das Ventilgehäuse zwei Gehäuseteile, wobei die Membran zwischen den Gehäuseteilen angeordnet ist. Das erste Gehäuseteil kann als Oberschale ausgestaltet sein und das zweite Gehäuseteil den Ventilsitz bzw. die Ventilsitzfläche aufweisen. Mit Vorteil ist in einem jeweiligen Gehäuseteil ein jeweiliger Strömungspfad ausgebildet. Die Gehäuseteile können zusammen einen ringförmigen Kederaufnahmeraum begrenzen, in dem die Membran mit ihrem Keder einsetzbar ist.

Vorrichtungstechnisch einfach kann der erste Strömungspfad im ersten Gehäuseteil einfach als im Wesentlichen gerader Kanalabschnitt bzw. Kanalbohrung ausgebildet sein, wobei der Kanalabschnitt das Ventilgehäuse vollständig durchsetzt und einerseits mit dem ersten Eingangsanschluss und andererseits mit dem ersten Ausgangsanschluss verbunden ist. Radial zum Kanalabschnitt kann die Fluidkammer ausgebildet sein, die von der Membran begrenzt ist. Bei der Herstellung des Gehäuseteils kann die Fluidkammer somit einfach von einer Seitenfläche des Gehäuseteils her in diese eingebracht werden. Die Fluidkammer ist beispielsweise ausgehend von dem Kanalabschnitt des ersten Strömungspfads kreiszylindrisch oder zylindrisch ausgebildet. Ein vom Kanalabschnitt entfernter Endabschnitt der Fluidkammer hat vorzugsweise eine kegelstumpfförmige Innenmantelfläche, die sich hin zur Membran verbreitert. Vorzugsweise entspricht ein maximaler Durchmesser der Fluidkammer des ersten Strömungspfads einem maximalen Durchmesser der Fluidkammer des zweiten Strömungspfads, wobei die Fluidkammern koaxial zueinander angeordnet sind. Hierdurch kann die dazwischen angeordnete Membran auf einfache Weise auf beiden Seiten gleich große Druckangriffsflächen aufweisen.

Zum Verbinden der Gehäuseteile kann einer der Gehäuseteile einen Gehäusevorsprung aufweisen, der in eine Gehäuseausnehmung des anderen Gehäuseteils eintaucht. Vorzugsweise hat der Gehäusevorsprung eine Außenmantelfläche mit einem etwa zylindrischen oder kreiszylindrischen Querschnitt. Des Weiteren kann der Gehäusevorsprung insbesondere koaxial von der Fluidkammer des ersten Strömungspfads oder alternativ des zweiten Strömungspfads durchsetzt sein. Somit ist der Vorsprung ringförmig ausgestaltet. Eine Innenmantelfläche der Gehäuseausnehmung ist dann an den Gehäusevorsprung angepasst und hat somit ebenfalls einen zylindrischen oder kreiszylindrische Querschnitt. In weiterer Ausgestaltung hat eine ringförmige Stirnfläche des Gehäusevorsprungs und eine ringförmige Bodenfläche der Gehäuseausnehmung jeweils eine Ringnut, die zusammen den Kederaufnahmeraum begrenzen. Der darin eingesetzte Keder der Membran kann dann auf einfache Weise zusätzlich als Dichtring wirken.

Die Anschlüsse der Strömungspfade sind vorzugsweise als Schlauchtüllen ausgebildet, die mit dem Ventilgehäuse verschraubt sind.

Erfindungsgemäß ist ein Dialysegerät mit einem Ventil gemäß einem der vorhergehenden Aspekte vorgesehen. Das Dialysegerät kann eine Pumpe aufweisen mit der frische Dialysierflüssigkeit von einer Dialysierflüssigkeitsquelle zu einem Dialysator über eine Bilanzierungseinrichtung förderbar ist. Über die gleiche oder eine weitere Pumpe ist die verbrauchte Dialysierflüssigkeit vom Dialysator über die Bilanzierungseinrichtung zu einer Dialysierflüssigkeitssenke förderbar. Vorzugsweise ist der erste Strömungspfad des Ventils an einen Strömungspfad mit der frischen Dialysierflüssigkeit und der zweite Strömungspfad des Ventils an einen Strömungspfad mit der verbrauchten Dialysierflüssigkeit angeschlossen.

Diese Lösung hat den Vorteil, dass eine Veränderung von Drücken in den Strömungspfaden durch das Ventil ausgleichbar ist und somit eine von der Bilanzierungseinrichtung vorgegebene Ultrafiltrationsmenge im Wesentlichen gleich bleibt. Somit ist die aus der Bilanzierungseinrichtung ausströmende Dialysierflüssigkeit gleich der zurückgeführten Dialysierflüssigkeit. Würde die Druckdifferenz nicht ausgeglichen werden, so könnte ab einer bestimmten Druckdifferenz zwischen den Strömungspfaden mit der frischen Dialysierflüssigkeit und der verbrauchten Dialysierflüssigkeit - bedingt durch eine Veränderung einer Ausdehnung der Bauteile des Dialysegeräts - eine Bilanz der Bilanzierungseinrichtung außerhalb einer zulässigen Toleranzgrenze liegen.

In weiterer Ausgestaltung der Erfindung kann der erste Strömungspfad des Ventils fluidisch zwischen der Bilanzierungseinrichtung und dem Dialysator an den Strömungspfad mit der frischen Dialysierflüssigkeit angeschlossen sein. Der zweite Strömungspfad des Fluids ist vorzugsweise fluidisch zwischen der Bilanzierungseinrichtung und der Druckmittelsenke an den Strömungspfad mit der verbrauchten Dialysierflüssigkeit angeschlossen.

Stromabwärts des Ventils im Strömungspfad mit der frischen Dialysierflüssigkeit kann ein (erstes) Konstantdruckventil angeordnet sein. Zusätzlich oder alternativ kann stromabwärts des Ventils im Strömungspfad mit der verbrauchten Dialysierflüssigkeit ein (zweites) Konstantdruckventil vorgesehen sein. Der Einsatz der Konstantdruckventile führt dazu, dass konstante Druckverhältnisse vorliegen. Die Kombination eines oder beider Konstantdruckventile mit dem erfindungsgemäßen Ventil führt dazu, dass ein konstanter Druck bzw. Vordruck für die Pumpe oder die Pumpen einstellbar ist, wodurch eine Ultrafiltrationsgenauigkeit erhöht ist.

Die erste Pumpe ist vorzugsweise stromaufwärts der Bilanzierungseinrichtung im Strömungspfad mit der frischen Dialysierflüssigkeit und die zweite Pumpe fluidisch zwischen der Bilanzierungseinrichtung und dem Dialysator in dem Strömungspfad mit der verbrauchten Dialysierflüssigkeit angeordnet. Das erste Konstantdruckventil kann dann im Strömungspfad der frischen Dialysierflüssigkeit zwischen dem Ventil und dem Dialysator und das zweite Konstantdruckventil im Strömungspfad der verbrauchten Dialysierflüssigkeit stromabwärts des Ventils vorgesehen sein. Im Strömungspfad mit der frischen Dialysierflüssigkeit kann zwischen dem ersten Konstantdruckventil und dem Ventil ein Dialysierflüssigkeitsfilter vorgesehen sein.

In weiterer Ausgestaltung der Erfindung kann eine Messung und Regelung der Ultrafiltrationsmenge über die Bilanzierungseinrichtung und einer Ultrafiltrationspumpe erfolgen. Die Ultrafiltrationspumpe ist eingangsseitig vorzugsweise zwischen der zweiten Pumpe und der Bilanzierungseinrichtung und ausgangsseitig vorzugsweise zwischen der Bilanzierungseinrichtung und dem Ventil angeschlossen (oder alternativ stromabwärts des Ventils), womit eine von der Ultrafiltrationspumpe geförderte Dialysierflüssigkeit die Bilanzierungseinrichtung umgeht.

Die Bilanzierungseinrichtung weist vorzugsweise zumindest eine Bilanzierungskammer mit zwei Zellen auf, die durch eine Membran voneinander getrennt sein können. Die eine Zelle ist hierbei vorzugsweise mit dem Strömungspfad mit der frischen Dialysierflüssigkeit und die andere Zelle mit dem Strömungspfad mit der verbrauchten Dialysierflüssigkeit verbunden. Mit Vorteil sind zwei Bilanzierungskammern mit jeweils zwei Zellen vorgesehen. Eine Beschickung und Entlassung von Dialysierflüssigkeit kann bei einer oder mehreren Bilanzierungskammern über Schaltventile gesteuert werden, die insbesondere elektromagnetisch betätigbar sind.

Im Folgenden wird eine bevorzugte Ausführungsform der Erfindung anhand von Zeichnungen näher erläutert. Es zeigen:
Figur 1 einen Schaltplan eines erfindungsgemäßen Dialysegeräts gemäß einer Ausführungsform,
Figur 2 einen Querschnitt eines erfindungsgemäßen Ventils des Dialysegeräts gemäß der Ausführungsform,
Figur 3 einen Querschnitt eines Ausgleichselements des Ventils,
Figur 4a und 4b jeweils einen Querschnitt eines Gehäuseteils des Ventils,
Figur 5 einen Vergleich von Druckdifferenzen des erfindungsgemäßen Dialysegeräts gegenüber dem Stand der Technik und
Figur 6a und 6b einen Vergleich von Abweichungen bei einer Ultrafiltrationsmenge zwischen dem erfindungsgemäßen Dialysegerät und dem Stand der Technik.

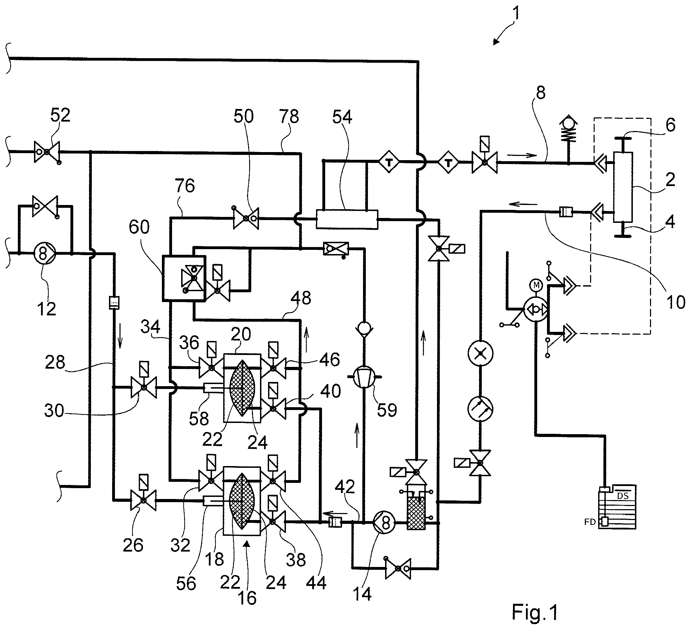

Gemäß Figur 1 ist ein Dialysegerät 1 mit einem Dialysator 2 dargestellt. Da der Grundaufbau des Dialysegeräts 1 hinlänglich bekannt ist, wird im Folgenden nur das für die Erfindung Wesentliche erläutert.

Mit dem Dialysegerät 1 wird Blut eines Patienten gereinigt, dass über eine Blutzuführleitung 4 zum Dialysator 2 geführt wird und über eine Blutabführleitung 6 von diesem abgeführt wird. Frische Dialysierflüssigkeit wird zum Dialysator 2 über eine Zuführleitung 8 zugeführt und verbrauchte Dialysierflüssigkeit vom Dialysator 2 über eine Abführleitung 10 abgeführt. Zum Fördern der Dialysierflüssigkeit zum Dialysator 2 ist eine erste Pumpe (Flusspumpe) vorgesehen. Diese fördert eine Dialysierflüssigkeit von einer nicht dargestellten Dialysierflüssigkeitsquelle über die Zuführleitung 8 zum Dialysator 2. Zum Abführen der verbrauchten Dialysierflüssigkeit vom Dialysator 2 ist eine zweite Pumpe 14 (Flusspumpe) vorgesehen. Die Pumpen 12 und 14 sind beispielsweise als Zahnradpumpen ausgestaltet. Im Strömungspfad mit der frischen Dialysierflüssigkeit ist zwischen der ersten Pumpe 12 und dem Dialysator 2 eine Bilanzierungseinrichtung 16 vorgesehen. Diese ist des Weiteren im Strömungspfad mit der verbrauchten Dialysierflüssigkeit zwischen der zweiten Pumpe 14 und einer in der Figur 1 nicht dargestellten Druckmittelsenke geschaltet.

Die Bilanzierungseinrichtung 16 hat eine erste Bilanzierungskammer 18 und eine zweite Bilanzierungskammer 20. Die Bilanzierungskammern 18 und 20 weisen jeweils eine erste und zweite Zelle 22 und 24 auf, die über eine Membran voneinander getrennt sind. Die Bilanzierungskammern 18 und 20 sind identisch ausgestaltet. Die darin vorgesehene Membran ist beidseitig flexibel auslenkbar. Die erste Zelle 22 der ersten Bilanzierungskammer 18 ist über ein erstes Schaltventil 26 mit einer Pumpenleitung 28 verbunden, die ausgangsseitig an die erste Pumpe 12 angeschlossen ist. Das Gleiche gilt für die erste Zelle 22 der zweiten Bilanzierungskammer 20, die über ein zweites Schaltventil 30 mit der Pumpenleitung 28 verbunden ist. Ausgangsseitig ist die erste Zelle 22 der ersten Bilanzierungskammer 18 über ein Schaltventil 32 mit einer Zuführleitung 34 verbunden, an die auch die erste Zelle 22 der zweiten Bilanzierungskammer 20 über ein Schaltventil 36 angeschlossen ist. Die ersten Zellen 22 sind somit mit dem Strömungspfad mit frischer Dialysierflüssigkeit verbunden. Dagegen sind die zweiten Zellen 24 der Bilanzierungskammer 18 und 20 mit dem Strömungspfad mit verbrauchter Dialysierflüssigkeit verbunden. Hierzu ist eine jeweilige zweite Zelle 24 über ein Schaltventil 38 beziehungsweise 40 mit einer Pumpenleitung 42 verbunden, die ausgangsseitig der zweiten Pumpe 14 angeschlossen ist. Über ein jeweiliges Schaltventil 44, 46 ist eine jeweilige zweite Zelle 24 mit einer Abführleitung 48 verbunden, die zur Dialysierflüssigkeitssenke führt. Die Schaltventile 30, 32, 36, 38, 40, 44, 46 sind elektromagnetisch betätigbar.

Im Folgenden ist die Funktionsweise der Bilanzierungseinrichtung 16 erläutert. Initial (vor Behandlungsbeginn) werden die Drehzahlen der Pumpen 12 und 14 aneinander angeglichen, womit ein Gleichlauf der Membranen der Bilanzierungskammern 18 und 20 zunächst sichergestellt ist. In einem ersten Schritt sind die Schaltventile 26, 36, 40, 44 aufgesteuert und die Schaltventile 30, 32, 38, 46 zugesteuert. Frische Dialysierflüssigkeit fliest somit von der ersten Pumpe 12 in die erste Zelle 22 der ersten Bilanzierungskammer 18 und verdrängt über die darin angeordnete Membran verbrauchte Dialysierflüssigkeit zur Dialysierflüssigkeitssenke. Zugleich fliest verbrauchte Dialysierflüssigkeit von der Pumpe 14 in die zweite Zelle 24 der zweiten Bilanzierungskammer 20, womit frische Dialysierflüssigkeit in der ersten Zelle 22 in Richtung des Dialysators 2 verdrängt wird. Im nächsten Schritt werden alle Schaltventile 30, 32, 36, 38, 40, 44, 46 geschlossen. Im Anschluss daran im folgenden Schritt sind die Schaltventile 30, 32, 38, 46 aufgesteuert und die Schaltventile 26, 36, 40, 44 zugesteuert. Somit fliest frische Dialysierflüssigkeit von der ersten Pumpe 12 in die erste Zelle 22 der zweiten Bilanzierungskammer 20 und verdrängt verbrauchte Dialysierflüssigkeit in der zweiten Zell 24 über die Abführleitung 48 zur Dialysierflüssigkeitssenke. Zugleich fliest verbrauchte Dialysierflüssigkeit von der zweiten Pumpe 14 in die zweite Zelle 24 der ersten Bilanzierungskammer 18 und verdrängt frische Dialysierflüssigkeit hin zum Dialysator 2. Anschließend werden alle Schaltventile 30, 32, 36, 38, 40, 44, 46 geschlossen und die Schritte wiederholt ausgeführt.

Um einen Druck zwischen der Bilanzierungseinrichtung 16 und dem Dialysator 2 im Strömungspfad mit der frischen Dialysierflüssigkeit konstant zu halten ist ein Konstantdruckventil 50 vorgesehen. Ein weiteres Konstantdruckventil 52 ist im Strömungspfad mit der verbrauchten Dialysierflüssigkeit stromabwärts der Bilanzierungseinrichtung 16 angeordnet. Zum Filtern der frischen Dialysierflüssigkeit ist zwischen der Bilanzierungseinrichtung 16 und dem Dialysator 2 des Weiteren ein Dialysierflüssigkeitsfilter 54 angeordnet. Dieser ist insbesondere stromabwärts des Konstantendruckventils 50 vorgesehen.

Eine Position der Membran einer jeweiligen Bilanzierungskammer 28 ist über einen jeweiligen induktiven Membranstellungssensor 56, 58 erfassbar. Ein jeweiliger Membranstellungssensor 56, 58 ist hierbei mit einer jeweiligen Membran verbunden und hat ein Sensorelement, das in einer Spule zur induktiven Positionsbestimmung bewegbar ist.

Über eine Ultrafiltrationspumpe 59 kann ein Teil der verbrauchten Dialysierflüssigkeit zwischen der zweiten Pumpe 14 und der Bilanzierungseinrichtung 16 abgezweigt und zur Dialysierflüssigkeitssenke unter Umgehung der Bilanzierungseinrichtung 16 gefördert werden. Die hierbei abgeführte Flüssigkeitsmenge entspricht beispielsweise der im Dialysator 2 vom Blut in die Dialysierflüssigkeit übergehenden Flüssigkeitsmenge.

Die Konstantdruckventile 50, 52 dienen dazu, dass die Pumpen 12 und 14 die Membranen der Bilanzierungskammern 18 und 20 gegen definierte Strömungswiderstände bewegen und Gegendrücke in beiden Bilanzierungskammern 18 und 20 identisch sind. Werden die Drehzahlen der Pumpen 12 und 14 zum Verändern eines Volumenstroms der Dialysierflüssigkeit verändert, so ändert sich hierdurch insbesondere auch ein Strömungswiderstand des Dialysierflüssigkeitsfilters 54. Bei unveränderter Einstellung der Konstantdruckventile 50 und 52 verändern sich somit die Gegendrücke der Bilanzierungskammern 18 und 20. Aufgrund der sich ändernden Drücke ändert sich auch eine Ausdehnung der Leitungen bzw. Schläuche des Dialysegeräts und der Bauteile der Bilanzierungseinrichtung 16, womit die Bilanz der Dialysierflüssigkeit der Bilanzierungseinrichtung 16 druckabhängig ist. Die Gegendrücke der beiden Bilanzierungskammern 18 und 20 können sich auch aufgrund einer Zusetzung des Dialysierflüssigkeitsfilters 54 ändern. Durch die Veränderung der Gegendrücke der Bilanzierungskammern 18 und 20, bei verschiedenen Betriebszuständen, kann es zu erheblichen Abweichungen der Ultrafiltrationsmengen kommen. Zum Ausgleich der Druckunterschiede zwischen dem Strömungspfad mit der frischen Dialysierflüssigkeit (Dialysierflüssigkeits-Zuführung) und dem Strömungspfad mit der verbrauchten Dialysierflüssigkeit (Dialysierflüssigkeits-Rückführung) ist das erfindungsgemäße Ventil bzw. Druckausgleichsventil 60 vorgesehen. Dieses ist zum Einen an dem Strömungspfad mit der frischen Dialysierflüssigkeit zwischen der Bilanzierungseinrichtung 16 und dem Konstantdruckventil 50 und zum Anderen an dem Strömungspfad mit der verbrauchten Dialysierflüssigkeit zwischen der Bilanzierungseinrichtung 16 und dem Konstantdruckventil 52 angeschlossen.

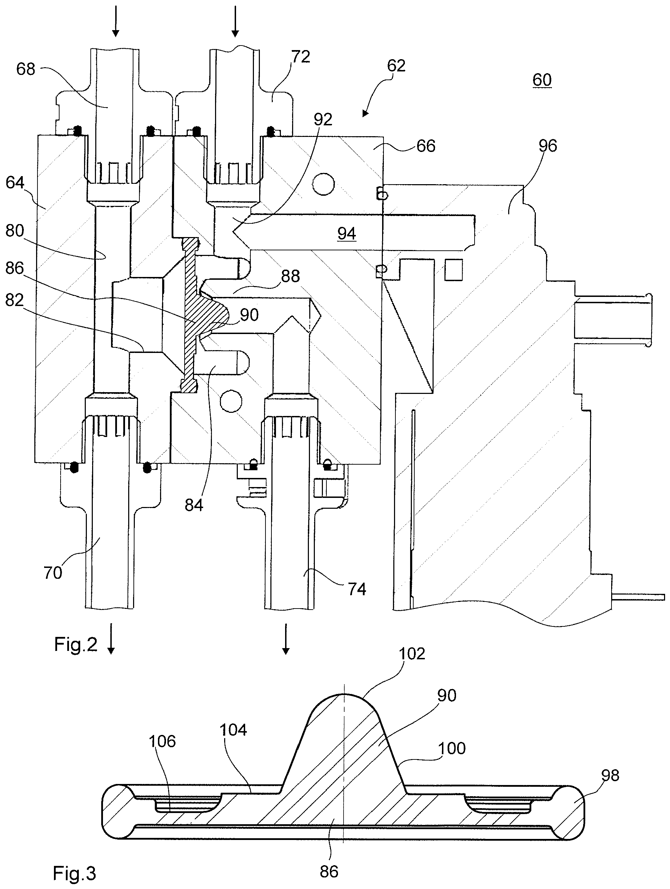

Gemäß Figur 2 ist das Druckausgleichsventil 60 dargestellt, das ein Ventilgehäuse 62 mit einem ersten Gehäuseteil 64 und einem zweiten Gehäuseteil 66 aufweist. Der erste Gehäuseteil 64 hat einen ersten Eingangsanschluss 68 und einen ersten Ausgangsanschluss 70. Der zweite Gehäuseteil 66 weist einen zweiten Eingangsanschluss 72 und einen zweiten Ausgangsanschluss 74 auf. Die Anschlüsse 68 bis 74 sind als Schlauchtüllen ausgebildet, die mit dem Ventilgehäuse 62 verschraubt sind. Der erste Eingangsanschluss 68 ist mit der Zuführleitung 34, siehe auch Figur 1, verbunden. Der Ausgangsanschluss 70 ist über eine Zwischenleitung 76 mit dem Konstantdruckventil 50 verbunden. Dagegen ist der Eingangsanschluss 72 mit der Abführleitung 48 und der Ausgangsanschluss 74 über eine Zwischenleitung 78 mit dem zweiten Konstantdruckventil 52 verbunden. Somit ist in dem ersten Ventilgehäuse 62 ein erster Strömungspfad für die frische Dialysierflüssigkeit und in dem zweiten Gehäuseteil 66 ein zweiter Strömungspfad für die verbrauchte Dialysierflüssigkeit ausgebildet. Zum Ausbilden des ersten Strömungspfads ist das erste Gehäuseteil 64 von einer Bohrung 80 durchsetzt. Radial zu dieser ist eine Fluidkammer 82 in den ersten Gehäuseteil 64 eingebracht, die sich hin zum zweiten Gehäuseteil 66 erstreckt. Das zweite Gehäuseteil 66 weist ebenfalls eine Fluidkammer 84 auf, die sich ausgehend von der Fluidkammer 82 erstreckt. Die Fluidkammern 82, 84 sind hierbei von einem Ausgleichselement in Form einer Membran 86 fluidisch voneinander getrennt. Die Membran 86 ist zwischen den Gehäuseteilen 64 und 66 lagefixiert. In die Fluidkammer 84 mündet etwa koaxial zur Membran 86 ein Kanalabschnitt 88. Dieser kragt abschnittsweise in die Fluidkammer 84 ein und begrenzt darin einen Ringraum. In den Kanalabschnitt 88 taucht die Membran 86 mit einem kegelförmigen Membranvorsprung 90 ein. Der Kanalabschnitt 88 ist durch eine Sacklochbohrung gebildet, in der eine weitere Sacklochbohrung etwa radial dazu einmündet, die mit dem zweiten Ausgangsanschluss 74 verbunden ist. Radial in die Fluidkammer 84 mündet eine Bohrung 92, die mit dem zweiten Eingangsanschluss 72 verbunden ist. Von dieser zweigt ein Bypass-Kanal in Form von einer Bohrung 94 ab, die von außen her in den zweiten Gehäuseteil 66 eingebracht ist und zum Anschluss eines Bypass-Ventils 96 dient. Verbrauchte Dialysierflüssigkeit strömt somit über den zweiten Eingangsanschluss 72, die Bohrung 92 in die Fluidkammer 84 und von dieser über den Kanalabschnitt 88 zum zweiten Ausgangsanschluss 74.

Gemäß Figur 3 ist die Membran 86 rotationssymmetrisch mit einem etwa kreiszylindrischen Querschnitt ausgebildet. Umfangsseitig weist sie einen Keder 98 auf, über den sie zwischen den Gehäuseteilen 64 und 66 festlegbar ist und der zusätzlich als Dichtelement dient. Der Membranvorsprung 90 ist koaxial zur Membran 86 ausgebildet und hat eine kegelstumpfförmige Außenmantelfläche 100 und eine abgerundete Stirnfläche 102. In Radialrichtung gesehen schließt sich an den Membranvorsprung 90 eine Ringfläche als Anschlagsschulter 104 an, die stirnseitig an den Kanalabschnitt 88, siehe auch Figur 2, anschlagen kann, um den Kanalabschnitt 88 zu verschließen, was untenstehend näher erläutert ist. Im Anschluss an die Anschlagschulter 104, in Radialrichtung gesehen, ist eine Ringnut 106 eingebracht, durch die eine Wandungsdicke der Membran 86 zur Erhöhung einer Flexibilität verringert ist.

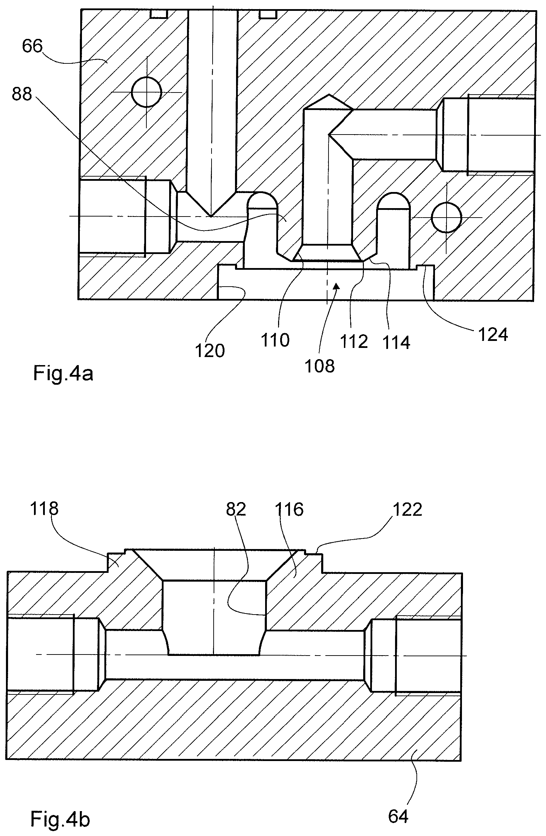

Gemäß Figur 4a hat der Kanalabschnitt 88 des ersten Gehäuseteils 64 eine Mündungsöffnung 108 mit einer kegelstumpfförmigen Innenmantelfläche 110, die sich in einer Richtung weg von der Membran 86, siehe Figur 2, verjüngt. Stirnseitig des Kanalabschnitts 88 schließt sich an die Mündungsöffnung 108 eine ringförmige Ventilsitzfläche 112 an, an die die Membran 86 mit der Anschlagschulter 104, siehe Figur 3, anlegbar ist. Die sich radial an die Ventilsitzfläche anschließende Umfangsfläche 114 ist kegelstumpfförmig ausgestaltet und verbreitert sich in einer Richtung weg von der Membran 86, siehe Figur 2.

Gemäß Figur 4b ist die Fluidkammer 82 des ersten Gehäuseteils 64 als Radialbohrung ausgebildet und hat etwa einen doppelt so großen Durchmesser wie die Bohrung 80, siehe Figur 2. Hin zum zweiten Gehäuseteil 64 verbreitert sich die Fluidkammer 82 mit einer kegelstumpfförmigen Innenmantelfläche 16. Das erste Gehäuseteil 64 hat des Weiteren einen einen etwa kreiszylindrischen Querschnitt aufweisenden Gehäusevorsprung 118, der koaxial von der Fluidkammer 82 durchsetzt ist. Dieser taucht in zusammengefügtem Zustand der Gehäuseteile 64 und 66 in eine Gehäuseausnehmung 120 des zweiten Gehäuseteils 66 ein. Stirnseitig des Gehäusevorsprungs 118 ist eine Ringnut 122 eingebracht. Des Weiteren ist bodenseitig der Gehäuseausnehmung 120 eine weitere Ringnut 124 eingebracht. Diese begrenzen zusammen einen Kederaufnahmeraum für den Keder 98 der Membran 86, siehe Figur 3. In zusammengefügtem Zustand der Gehäuseteile 64 und 66 ist die Stirnfläche des Gehäusevorsprungs 118 von der Bodenfläche der Gehäuseausnehmung 120 beabstandet, wobei zwischen diesen ein Ringspalt gebildet ist, über den die Membran 86 aus dem Kederaufnahmeraum herausgeführt ist.

Im zusammengesetzten Zustand der Gehäuseteile 64 und 66 werden diese vorzugsweise verschraubt. Das Bypass-Ventil 96 ist ebenfalls vorzugsweise mit dem Ventilgehäuse 62 verschraubt.

Im Folgenden wird die Funktionsweise des Druckausgleichsventils 60 anhand der Figuren 2 bis 4b näher erläutert. Steigt ein Druck im ersten Strömungspfad des ersten Gehäuseteils 64, so wird die Membran 86 gemäß Figur 2 aus der darin gezeigten Neutralstellung hin in Richtung des Kanalabschnitts 88 ausgelenkt. Hierdurch taucht zum Einen der Membranvorsprung 90 tiefer in den Kanalabschnitt 88 ein und zum Anderen nähert sich die Anschlagschulter 104, siehe Figur 3, der Ventilsitzfläche 112 an. Dies führt zu einer Verengung des Strömungsquerschnitts des zweiten Strömungspfads in dem zweiten Gehäuseteil 66, womit der Druck eingangsseitig des zweiten Gehäuseteils 62 ansteigt und somit gemäß Figur 1 ausgangsseitig der Bilanzierungseinrichtung 16 im Strömungspfad der verbrauchten Dialysierflüssigkeit. Sinkt der Druck im ersten Strömungspfad des ersten Gehäuseteils 64, so wird die Membran 86 in entgegengesetzter Richtung bewegt und der Druck im zweiten Strömungspfad passt sich entsprechend an.

Durch die Kombination der Konstantdruckventile 50, 52 mit dem Druckausgleichsventil 60 wird das Einstellen eines konstanten Drucks (Vordrucks) für die Pumpen 12 und 14 und die Ultrafiltrationspumpe 59 ermöglicht. Dies führt zu einer verbesserten Ultrafiltrationsgenauigkeit und einer verbesserten Substitutionsgenauigkeit bzw. einer genaueren Online-Infusionsbolusgabe - insbesondere durch das Konstantdruckventil 50 -, da die hierzu verwendete Schlauchpumpe (nicht dargestellt) eine größere Genauigkeit aufweist, wenn der Druck im ersten Strömungspfad mit der frischen Dialysierflüssigkeit in einem positiven Bereich konstant gehalten wird. Positiver Bereich bedeutet, dass der Druck eingangsseitig größer 0 ist. Dagegen stellt sich beispielsweise bei dem Dialysegerät der eingangs erläuterten Druckschrift EP 0 597 817 durch den Druckausgleich mittels zweier Druckausgleichsventile ein Druckniveau ein, das bestimmt wird von den Strömungswiderständen im Dialysierflüssigkeitskreislauf und deshalb nachteilig nicht zwingend zu einem konstanten positiven Druck führt. Druckunterschiede zwischen den Eingangsanschlüssen 68 und 72 des Druckausgleichsventils 60 aus Figur 2, die vor dem Druckausgleichsventil messbar sind, sind nach dem Durchströmen der Dialysierflüssigkeit durch die Fluidkammern 82 und 84 nicht mehr vorhanden.

Ein Flattern und Vibrieren der Membran 86 wird durch die kegelstumpfförmige Ausgestaltung des Membranvorsprungs 90 und die vorteilhafte Anströmung der Membran 86 vollständig eliminiert.

Mit dem Druckausgleichsventil 60 ist eine Kalibrierung der Konstantdruckventile 50 und 52 nicht mehr notwendig.

Durch die Anpassung der Druckunterschiede durch das Druckausgleichsventil 60 kann eine größere Ausdehnung der Bilanzkammern 18 und 20 zugelassen werden, ohne dass hierdurch die Ultrafiltrationsgenauigkeit leidet. Die Bilanzkammern 18 und 20 können somit beispielsweise aus einem weicheren Material bestehen und/oder dünner ausgebildet sein und/oder weniger Verstrebungen aufweisen. Die mit Druck beaufschlagten Bauteile des Dialysegeräts können des Weiteren auch kostengünstige Spritzgussteile sein. Außerdem können die Leitungen des Dialysegeräts 1 aus kostengünstigen, vergleichsweise dünnwandigen Silikonschläuchen bestehen, die beispielsweise eine Wandstärke zwischen 2 bis 2,5 mm aufweisen. Bisher haben Silikonschläuche, die für ein Dialysegerät eingesetzt werden, üblicherweise eine Wandstärke von 3mm.

Alternativ zu den kostenintensiven Konstantdruckventile 50 und 52 ist alternativ denkbar, mechanisch hergestellte Drosselstellen, wie z. B. eine Verengung bzw. eine Durchmesserreduzierung in einer Bohrung oder in einem Schlauch vorzusehen.

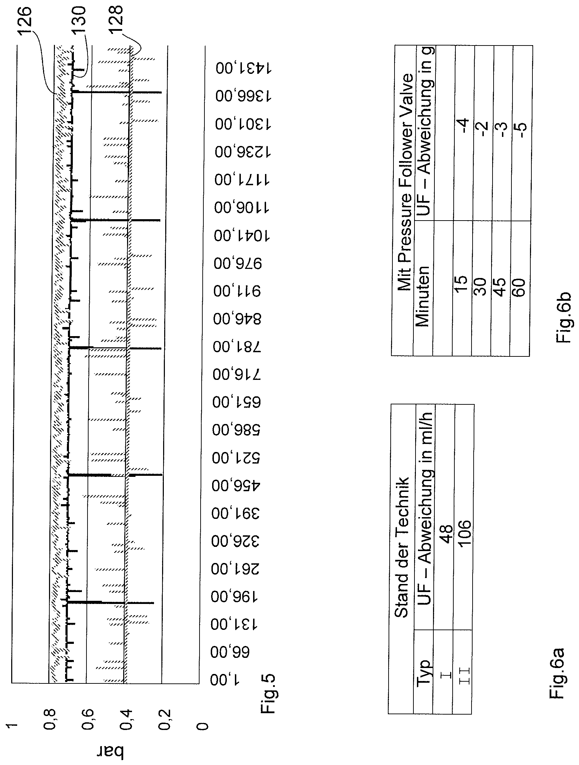

Gemäß Figur 5 ist eine Druckdifferenz 126 des erfindungsgemäßen Dialysegeräts 1 zwischen dem Strömungspfad mit der verbrauchten Dialysierflüssigkeit und dem Strömungspfad mit der frischen Dialysierflüssigkeit über die Zeit abgebildet. Es ist erkennbar, dass die Druckdifferenz 126 geringe Schwankungen aufweist. Dagegen sind bei einer Druckdifferenz 128 von einem Dialysegerät aus dem Stand der Technik große Schwankungen erkennbar. Des Weiteren ist der Figur 5 ein eingestellter Widerstandsbeiwert 130 entnehmbar.

Die Tabelle gemäß Figur 6a zeigt Mittelwerte, die für die mittlere Ultrafiltrationsmengen-(UF)-Abweichung (ml/Stunde) in Datenblättern bekannter Geräte (Typ I und Typ II) angegeben werden. Demnach ist die Abweichung der Ultrafiltrationsmenge bei bekannten Dialysegeräten mit steigender Betriebszeit relativ hoch. Dagegen bleibt gemäß Figur 6b die Ultrafiltrationsmenge bei dem erfindungsgemäßen Dialysegerät 1 über die Betriebszeit im Wesentlichen konstant.

Offenbart ist ein Druckausgleichsventil für ein Dialysegerät. Dieses hat ein Ventilgehäuse, in dem ein Strömungspfad mit frischer Dialysierflüssigkeit von einem Strömungspfad mit verbrauchter Dialysierflüssigkeit durch eine Membran getrennt ist. Die Membran und das Ventilgehäuse sind dabei derart ausgestaltet, dass ein Druckausgleich ermöglicht ist. Außerdem ist die Membran derart ausgestaltet, dass im Betrieb keine oder verminderte Vibrationen und/oder keine oder verminderte Geräuschemissionen auftreten.

Außerdem ist ein Dialysegerät mit einem derartigen Druckausgleichsventil offenbart. Diese hat eine Bilanzierungseinrichtung, wobei das Druckausgleichsventil ausgangsseitig der Bilanzierungseinrichtung angeordnet ist.

### Bezugszeichenliste:

- 1: Dialysegerät
- 2: Dialysator
- 4: Blutzuführleitung
- 6: Blutabführleitung
- 8: Zuführleitung
- 10: Abführleitung
- 12: erste Pumpe
- 14: zweite Pumpe
- 16: Bilanzierungseinrichtung
- 18: erste Bilanzierungskammer
- 20: zweite Bilanzierungskammer
- 22: erste Zelle
- 24: zweite Zelle
- 26: erstes Schaltventil
- 28: Pumpenleitung
- 30: Schaltventil
- 32: Schaltventil
- 34: Zuführleitung
- 36: Schaltventil
- 38: Schaltventil
- 40: Schaltventil
- 42: Pumpenleitung
- 44: Schaltventil
- 46: Schaltventil
- 48: Abführleitung
- 50: Konstantdruckventil
- 52: Konstantdruckventil
- 54: Dialysierflüssigkeitsfilter
- 56: Membranstellungssensor
- 58: Membranstellungssensor
- 59: Ultrafiltrationspumpe
- 60: Druckausgleichsventil
- 62: Ventilgehäuse
- 64: erster Gehäuseteil
- 66: zweiter Gehäuseteil
- 68: erster Eingangsanschluss
- 70: erster Ausgangsanschluss
- 72: zweiter Eingangsanschluss
- 74: zweiter Ausgangsanschluss
- 76: Zwischenleitung
- 78: Zwischenleitung
- 80: Bohrung
- 82: Fluidkammer
- 84: Fluidkammer
- 86: Membran
- 88: Kanalabschnitt
- 90: Membranvorsprung
- 92: Bohrung
- 94: Bohrung
- 96: Bypass-Ventil
- 98: Keder
- 100: Außenmantelfläche
- 102: Stirnfläche
- 104: Anschlagschulter
- 106: Ringnut
- 108: Mündungsöffnung
- 110: Innenmantelfläche
- 112: Ventilsitzfläche
- 114: Umfangsfläche
- 116: Innenmantelfläche
- 118: Gehäusevorsprung
- 120: Gehäuseausnehmung
- 122: Ringnut
- 124: Ringnut
- 126: Druckdifferenz
- 128: Druckdifferenz
- 130: Widerstandsbeiwert

## Patentansprüche

1. Ventil für ein Dialysegerät, mit einem Ventilgehäuse (62), in dem ein erster mit einem ersten Eingangsanschluss (68) und einem ersten Ausgangsanschluss (70) fluidisch verbundener Strömungspfad (80, 82) und ein zweiter mit einem zweiten Eingangsanschluss (72) und einem zweiten Ausgangsanschluss (74) fluidisch verbundener Strömungspfad (92, 84, 88) ausgebildet ist, wobei
in dem Ventilgehäuse (62) ein Ausgleichselement (86) zum Ausgleichen von Drücken der Strömungspfade (80, 82; 92, 84, 88) angeordnet ist, über das die Fluide eines jeweiligen Strömungspfads in Wirkverbindung stehen,
das Ausgleichselement (86) derart ausgestaltet ist, dass eine Vibration und/oder Geräuschemission vermindert ist, und/oder dass dem Ausgleichselement (86) ein Ventilsitz (112) zugeordnet ist,
das Ausgleichselement eine Membran (86) ist, die einerseits vom Fluid des ersten Strömungspfads (80, 82) und andererseits vom Fluid des zweiten Strömungspfads (92, 84, 88) beaufschlagt ist, und einen Membranvorsprung (90) aufweist, und
die Membran (86) als Dichtelement zum fluidischen Trennen beider Strömungspfade (80, 82; 92, 84, 88) ausgebildet ist,
**dadurch gekennzeichnet, dass**
der zweite Strömungspfad (92, 84, 88) einen Fluidraum (84) aufweist, in den ein Kanalabschnitt (88) des zweiten Strömungspfads mündet, wobei in den Kanalabschnitt der Membranvorsprung (90) eintauchbar ist.

2. Ventil nach Anspruch 1, wobei eine Mündungsöffnung (108) des Kanalabschnitts (88) zum Ausbilden des Ventilsitzes eine Ventilsitzfläche (112) aufweist.

3. Ventil nach Anspruch 2, wobei die Membran (86) eine Anschlagschulter zum Anliegen an die Ventilsitzfläche (112) hat.

4. Ventil nach einem der Ansprüche 1 bis 3, wobei der Membranvorsprung (90) kegelförmig oder kegelstumpfförmig ausgebildet ist und sich in einer Richtung weg von der Membran (86) verjüngt.

5. Ventil nach einem der Ansprüche 1 bis 4, wobei der Kanalabschnitt (88) als Vorsprung in den Fluidraum (84) mündet und darin einen Ringraum begrenzt.

6. Ventil nach einem der Ansprüche 1 bis 5, wobei ein Bypass-Kanal (94) zum Verbinden mit einem Bypass-Ventil (96) vom zweiten Strömungspfad im zweiten Gehäuseteil (66) abzweigt.

7. Ventil nach einem der Ansprüche 1 bis 6, wobei der erste Strömungspfad (80, 82) einen Fluidraum (82) aufweist, der von der Membran (86) begrenzt ist.

8. Ventil nach einem der Ansprüche 1 bis 7, wobei das Ventilgehäuse (62) mit zwei Gehäuseteilen (64, 66) ausgebildet ist, und die Membran (86) zwischen den Gehäuseteilen (64, 66) angeordnet ist.

9. Ventil nach Anspruch 8, wobei zum Verbinden der Gehäuseteile (64, 66) ein Gehäuseteil (64) einen Gehäusevorsprung (118) hat, der in eine Gehäuseausnehmung (120) des anderen Gehäuseteils (66) eintaucht.

10. Dialysegerät mit einem Ventil gemäß einem der vorhergehenden Ansprüche, mit einer Pumpe (12) zum Fördern einer frischen Dialysierflüssigkeit von einer Dialysierflüssigkeitsquelle zu einem Dialysator (2) über eine Bilanzierungseinrichtung (16), wobei verbrauchte Dialysierflüssigkeit von dem Dialysator (2) über die Bilanzierungseinrichtung (16) zu einer Dialysatorsenke förderbar ist, wobei der erste Strömungspfad (80, 82) des Ventils (60) an den Strömungspfad mit der frischen Dialysierflüssigkeit und der zweite Strömungspfad (92, 84, 88) des Ventils (60) an den Strömungspfad mit der verbrauchten Dialysierflüssigkeit angeschlossen ist.

11. Dialysegerät nach Anspruch 10, wobei das Ventil (60) stromabwärts der Bilanzierungseinrichtung (16) angeordnet ist.

12. Dialysegerät nach Anspruch 10 oder 11, wobei stromabwärts des Ventils (60) im Strömungspfad mit der frischen Dialysierflüssigkeit ein Konstantdruckventil (50) angeordnet ist, und/oder wobei stromabwärts des Ventils (60) im Strömungspfad mit der verbrauchten Dialysierflüssigkeit ein Konstantdruckventil (52) angeordnet ist.

## Claims

1. A valve for a dialysis machine, comprising a valve housing (62) having formed therein a first flow path (80, 82) in fluid communication with a first inlet connection (68) and a first outlet connection (70) and a second flow path (92, 84, 88) in fluid communication with a second inlet connection (72) and a second outlet connection (74),
said valve housing (62) having arranged therein an equalizer element (86) which equalizes pressures of the flow paths (80, 82; 92, 84, 88) and through which the fluids of respective flow paths operatively interact, wherein
the equalizer element (86) is configured such that vibration and/or noise emission will be reduced, and/or that the equalizer element (86) has associated therewith a valve seat (112),
the equalizer element is a diaphragm (86), which is acted upon by the fluid of the first flow path (80, 82) on the one hand and by the fluid of the second flow path (92, 84, 88) on the other and which is provided with a diaphragm projection (90), and
the diaphragm (86) is configured as a sealing element for fluidically separating the two flow paths (80, 82; 92, 84, 88),
**characterized in that**
the second flow path (92, 84, 88) comprises a fluid space (84) into which a channel section (88) of the second flow path opens, the diaphragm projection (90) being adapted to be introduced into said channel section.

2. The valve according to claim 1, wherein an outlet opening (108) of the channel section (88) is provided with a valve mating surface (112) so as to define the valve seat.

3. The valve according to claim 2, wherein the diaphragm (86) has a stop shoulder for contacting the valve mating surface (112).

4. The valve according to one of the claims 1 to 3, wherein the diaphragm projection (90) is configured to be conical or frusto-conical in shape and tapers in a direction away from the diaphragm (86).

5. The valve according to one of the claims 1 to 4, wherein the channel section (88) ends in the form of a protrusion within the fluid chamber (84) and defines an annular space therein.

6. The valve according to one of the claims 1 to 5, wherein a bypass channel (94) for connection to a bypass valve (96) branches off from the second flow path in the second housing part (66).

7. The valve according to one of the claims 1 to 6, wherein the first flow path (80, 82) comprises a fluid chamber (82), which is delimited by the diaphragm (86).

8. The valve according to one of the claims 1 to 7, wherein the valve housing (62) is configured such that it comprises two housing parts (64, 66), and the diaphragm (86) is arranged between these housing parts (64, 66).

9. The valve according to claim 8, wherein, for connecting the housing parts (64, 66), one housing part (64) is provided with a housing projection (118), which is introduced in a housing recess (120) of the other housing part (66).

10. A dialysis machine with a valve according to one of the preceding claims, comprising a pump (12) for conveying a fresh dialysate from a dialysate source to a dialyzer (2) via a balancing unit (16), wherein exhausted dialysate is conveyable from the dialyzer (2) via the balancing unit (16) to a dialysate sink, and wherein the first flow path (80, 82) of the valve (60) communicates with the flow path with fresh dialysate and the second flow path (92, 84, 88) of the valve (60) communicates with the flow path with exhausted dialysate.

11. The dialysis machine according to claim 10, wherein the valve (60) is arranged downstream of the balancing unit (16).

12. The dialysis machine according to claim 10 or 11, wherein a constant pressure valve (50) is arranged downstream of the valve (60) in the flow path with the fresh dialysate, and/or wherein a constant pressure valve (52) is arranged downstream of the valve (60) in the flow path with the exhausted dialysate.

## Revendications

1. Valve pour un appareil de dialyse, avec un boîtier de valve (62), dans lequel sont réalisés un premier trajet d'écoulement (80, 82) en communication fluidique avec un premier raccord d'entrée (68) et avec un premier raccord de sortie (70) et un deuxième trajet d'écoulement (92, 84, 88) en communication fluidique avec un deuxième raccord d'entrée (72) et avec un deuxième raccord de sortie (74), dans laquelle
un élément de compensation (86) servant à compenser des pressions des trajets d'écoulement (80, 82 ; 92, 84, 88) est disposé dans le boîtier de valve (62), par l'intermédiaire duquel les fluides d'un trajet d'écoulement respectif sont en liaison fonctionnelle,
l'élément de compensation (86) est configuré de telle manière qu'une vibration et/ou une émission de bruit sont atténuées et/ou qu'un siège de valve (112) est associé à l'élément de compensation (86),
l'élément de compensation est une membrane (86), qui est soumise d'une part à l'action du fluide du premier trajet d'écoulement (80, 82) et d'autre part à l'action du fluide du deuxième trajet d'écoulement (92, 84, 88), et présente une partie faisant saillie de membrane (90), et
la membrane (86) est réalisée sous la forme d'un élément étanche servant à séparer de manière fluidique les deux trajets d'écoulement (80, 82 ; 92, 84, 88),
**caractérisée en ce que**
le deuxième trajet d'écoulement (92, 84, 88) présente un espace de fluide (84), dans lequel débouche une section de canal (88) du deuxième trajet d'écoulement, dans laquelle la partie faisant saillie de membrane (90) peut être plongée dans la section de canal.

2. Valve selon la revendication 1, dans laquelle une ouverture d'embouchure (108) de la section de canal (88) présente, afin de réaliser le siège de valve, une surface de siège de valve (112).

3. Valve selon la revendication 2, dans laquelle la membrane (86) a un épaulement de butée destiné à reposer au niveau de la surface de siège de valve (112).

4. Valve selon l'une quelconque des revendications 1 à 3, dans laquelle la partie faisant saillie de membrane (90) est réalisée de manière à présenter une forme de cône ou une forme de cône tronqué et se rétrécit dans une direction s'éloignant de la membrane (86).

5. Valve selon l'une quelconque des revendications 1 à 4, dans laquelle la section de canal (88) débouche en tant que partie faisant saillie dans l'espace de fluide (84) et y délimite un espace annulaire.

6. Valve selon l'une quelconque des revendications 1 à 5, dans laquelle un canal de dérivation (94) destiné à être relié à une valve de dérivation (96) bifurque en partant du deuxième trajet d'écoulement dans la seconde partie de boîtier (66).

7. Valve selon l'une quelconque des revendications 1 à 6, dans laquelle le premier trajet d'écoulement (80, 82) présente un espace de fluide (82), qui est délimité par la membrane (86).

8. Valve selon l'une quelconque des revendications 1 à 7, dans laquelle le boîtier de valve (62) est réalisé avec deux parties de boîtier (64, 66) et la membrane (86) est disposée entre les parties de boîtier (64, 66).

9. Valve selon la revendication 8, dans laquelle afin de relier les parties de boîtier (64, 66), une partie de boîtier (64) a une partie faisant saillie de boîtier (118), qui plonge dans un évidement de boîtier (120) de l'autre partie de boîtier (66).

10. Appareil de dialyse avec une valve selon l'une quelconque des revendications précédentes, avec une pompe (12) servant à refouler un liquide de dialyse frais provenant d'une source de liquide de dialyse en direction d'un dialyseur (2) par l'intermédiaire d'un dispositif d'équilibrage (16), dans lequel du liquide de dialyse usagé peut être refoulé depuis le dialyseur (2) en direction d'un puits de dialyseur par l'intermédiaire du dispositif d'équilibrage (16), sans lequel le premier trajet d'écoulement (80, 82) de la valve (60) est raccordé au trajet d'écoulement présentant le liquide de dialyse frais et le deuxième trajet d'écoulement (92, 84, 88) de la valve (60) est raccordé au trajet d'écoulement présentant le liquide de dialyse usagé.

11. Appareil de dialyse selon la revendication 10, dans lequel la valve (60) est disposée en aval du dispositif d'équilibrage (16).

12. Appareil de dialyse selon la revendication 10 ou 11, dans lequel une valve de pression constante (50) est disposée en aval de la valve (60) dans le trajet d'écoulement présentant le liquide de dialyse frais, et/ou dans lequel une valve de pression constante (52) est disposée en aval de la valve (60) dans le trajet d'écoulement présentant le liquide de dialyse usagé.
